(19) Europäisches Patentamt
European Patent Office
Office européen des brevets

(11) EP 1 375 477 B1

# (12) EUROPÄISCHE PATENTSCHRIFT

(45) Veröffentlichungstag und Bekanntmachung des Hinweises auf die Patenterteilung:
**30.09.2009 Patentblatt 2009/40**

(51) Int Cl.:
*C07C 303/38 (2006.01)* *B01J 31/22 (2006.01)*

(21) Anmeldenummer: **03012670.0**

(22) Anmeldetag: **04.06.2003**

## (54) Verfahren zur Herstellung von mono-N-sulfonylierten Diaminen

Process for preparing mono-N-sulfonylated diamines

Procédé de préparation de diamines mono-N-sulfonylées

(84) Benannte Vertragsstaaten:
**AT BE BG CH CY CZ DE DK EE ES FI FR GB GR HU IE IT LI LU MC NL PT RO SE SI SK TR**

(30) Priorität: **17.06.2002 DE 10226944**

(43) Veröffentlichungstag der Anmeldung:
**02.01.2004 Patentblatt 2004/01**

(73) Patentinhaber: **Saltigo GmbH**
**40764 Langenfeld (DE)**

(72) Erfinder:
- **Bosch, Boris Elmar, Dr.**
  **50668 Köln (DE)**
- **Gerhartz, Frank**
  **51381 Leverkusen (DE)**

(74) Vertreter: **Wichmann, Birgid et al**
**LANXESS Deutschland GmbH**
**LIP-IPR**
**Q 18 / 1450**
**51369 Leverkusen (DE)**

(56) Entgegenhaltungen:
- **BALSELLS, JAUME ET AL.: J. ORG. CHEM., Bd. 65, 2000, Seiten 5005-5008, XP002261976**
- **BALSELLS, JAUME ET AL.: TETRAHEDRON-ASYMMETRY, Bd. 9, 1998, Seiten 4135-4142, XP0001091305**
- **NG K ET AL: "Synthesis of homochiral pentadentate sulfonamide-based ligands" TETRAHEDRON: ASYMMETRY, ELSEVIER SCIENCE PUBLISHERS, AMSTERDAM, NL, Bd. 12, Nr. 12, 16. Juli 2001 (2001-07-16), Seiten 1719-1722, XP004298252 ISSN: 0957-4166**
- **MEUZELAAR G ET AL: "Chemistry of Opium Alkaloids, 45. Improvements in the Total Synthesis of Morphine" EUROPEAN JOURNAL OF ORGANIC CHEMISTRY, WILEY-VCH VERLAG, WEINHEIM, DE, Bd. 9, 1999, Seiten 2315-2321, XP002192470 ISSN: 1434-193X**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 375 477 B1

Printed by Jouve, 75001 PARIS (FR)

**Beschreibung**

Die vorliegende Erfindung betrifft ein Verfahren zur Herstellung von mono-N-sulfonylierten Diaminen durch Umsetzung von Diaminen mit Sulfonylhalogeniden in Gegenwart von Wasser, Base und organischem Lösungsmittel.

Mono-N-sulfonylierte Diamine, insbesondere in optisch aktiver Form haben beispielsweise als Liganden in der Katalyse hohe technische Bedeutung erlangt (siehe zum Beispiel Noyori et al. J. Amer. Chem. Soc. 1995, 117, 7562).

Die Herstellung mono-N-sulfonylierter Diamine ist prinzipiell bekannt. So ist in R.A. Sheldon et al., Eur. J. Org. Chem. 1999, 2315 beispielsweise deren Herstellung aus Diaminen mit Sulfonylhalogeniden in Gegenwart von Triethylamin beschrieben.

BALSELLS, JAUME ET AL: J. ORG. CHEM., Bd.65, 2000, Seiten 5005-5008, beschreibt die Herstellung von Monosulfonamiden von 1,2-Diamincyclohexan, ausgehend von einer Lösung des Diamintartrates in wässriger Natronlauge durch Umsetzung von Sulfonylchlorid.

BALSELLS, JAUME ET AL.: TETRAHEDON-ASYMMETRY, Bd. 9, 1998, Seiten 4135-4142, beschreibt eine Lösung eines Monosulfonamids der Formel (I), die zur Herstellung eines Imins und letztlich zur Herstellung eines Katalysators verwendet wird.

MEUZELAAR G ET AL:: "Chemistry of Opium Alkaloids, 45. Improvements in the Total Synthesis of Morphine" EUROPEAN JOURNAL OF ORGANIC CHEMISTRY, WILEY-VCH VERLAG, WEINHEIM, DE, Bd. 9, 1999, Seiten 2315-2321, beschreibt Hydrierkatalysatoren, die durch Umsetzung von Rutheniumkomplexen der Formel (IV) mit Lösungen von Monosulfonamiden der Formel (I) erhalten werden.

Nachteilig an diesem Verfahren ist jedoch, dass üblicherweise Gemische von unsulfonierten, mono-N-sulfonierten und di-N-sulfonierten Produkten entstehen, die einer aufwändigen chromatographischen Trennung unterzogen werden müssen (siehe auch EP-A 1 174 426). Die Selektivitäten reichen bei den bekannten Verfahren von 30 bis 85 % bezogen auf das gewünschte mono-N-substitutierte Diamin, was für die technische Durchführung unbefriedigend ist.

Es bestand daher das Bedürfnis ein Verfahren bereitzustellen, das die Herstellung von mono-N-sulfonierten Diaminen in effizienter Weise mit guten Selektivitäten ermöglicht.

Es wurde nun ein Verfahren zur Herstellung von Verbindungen der Formel (I),

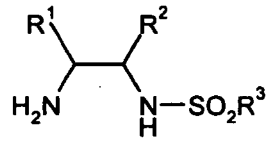

(I)

in der

$R^1$ und $R^2$ für Phenyl stehen und

$R^3$ für $C_1$-$C_4$-Alkyl, $C_1$-$C_4$-Fluoralkyl, Phenyl oder Naphthyl steht, das mit keinem, einem, zwei, drei, vier oder fünf Resten weiter substituiert sein kann, die ausgewählt sind aus der Gruppe $C_1$-$C_4$-Alkyl, $C_1$-$C_4$-Alkoxy, $C_1$-$C_4$-Fluoralkyl, Fluor und Chlor ,
dadurch gekennzeichnet, dass
Diamine der Formel (II)

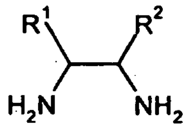

(II)

in der

$R^1$ und $R^2$ die unter der Formel (I) genannte Bedeutung besitzen

- in Gegenwart von Wasser und

- in Gegenwart von organischem Lösungsmittel, wobei und Volumenverhältnis von Wasser in organischen Lösungsmittel 20:1 bis 1:20 beträgt, und
- in Gegenwart von Basen aus der Gruppe Alkali- oder Erdalkalihydroxide oder -carbonate
- bei einer Temperatur im Bereich von -20°C bis 50°C mit Sulfonylhalogeniden der Formel (III) umgesetzt werden,

$$R^3SO_2X \qquad (III)$$

in der

X für Fluor, Chlor, Brom oder Iod steht und

$R^3$ die unter der Formel (I) genannte Bedeutung besitzt,

wobei das molare Verhältnis der Verbindungen (II) zu den Verbindungen der Formel (III) 0,8 bis 1,3 Äquivalente beträgt.

Die Verbindungen der Formel (I) und gegebenenfalls die Verbindungen der Formeln (II) und oder (III) sind chiral. Der Rahmen der Erfindung umfasst ausdrücklich sowohl die reinen Stereoisomeren (Enantiomeren und Diastereomeren) als auch beliebige Mischungen davon wie zum Beispiel Racemate.

$R^3$ steht besonders bevorzugt für Methyl, Trifluormethyl, Pentafluorethyl, Nonafluorbutyl, Phenyl, p-Tolyl, p-Ethylphenyl, p-Anisyl, p-Ethoxyphenyl, p-Chlorphenyl, 2,4,6-Trimethylphenyl, 2,4,6-Triisopropylphenyl, p-Fluorphenyl, Pentafluorphenyl und Naphthyl.

$R^3$ steht ganz besonders bevorzugt für p-Tolyl, Phenyl und Naphtyl, wobei p-Tolyl noch weiter bevorzugt ist.

X steht bevorzugt für Fluor oder Chlor, besonders bevorzugt für Chlor.

Bevorzugt werden chirale Verbindungen der Formel (II) eingesetzt, die eine optische Reinheit von 80 % ee oder mehr, besonders bevorzugt von 90 % ee oder mehr und ganz besonders bevorzugt von 98,5 % ee oder mehr aufweisen.

Die optische Reinheit ist dabei definiert als:

$$ee[S] = (m[S] - m[R])/m(S+R)$$

wobei
ee (S) die optische Reinheit des Enantiomers S, m(S) die Stoffmenge des Enantiomers S und m(R) die Stoffmenge des Enantiomers R ist. Die Angabe erfolgt üblicherweise in Prozent "enantiomeric excess" (% ee = ee/100).

Insbesondere ist das erfindungsgemäße Verfahren für die Herstellung folgender Verbindungen der Formel (II) geeignet:

N-[(1R,2R) und (1S,2S)-2-amino-1,2-diphenylethyl]-p-tolylsulfonamid, N-[(1R,2R) und (1S,2S)-2-amino-1,2-diphenylethyl]-o-tolylsulfonamid, N-[(1R,2R) und (1S,2S)-2-amino-1,2-diphenylethyl]-m-tolylsulfonamid, N-[(1R,2R) und (1S,2S)-2-amino-1,2-diphenylethyl]-phenylsulfonamid, N-[(1R,2R) und (1S,2S)-2-amino-1,2-diphenylethyl]-4-ethylphenylsulfonamid, N-[(1R,2R) und (1S,2S)-2-amino-1,2-diphenylethyl]-3-ethylphenylsulfonamid, N-[(1R,2R) und (1S,2S)-2-amino-1,2-diphenylethyl]-2-ethylphenylsulfonamid, N-[(1R,2R) und (1S,2S)-2-amino-1,2-diphenylethyl]-2,4,6-trimethylphenylsulfonamid, N-[(1R,2R) und (1S,2S)-2-amino-1,2-diphenylethyl]-2,4,6-triisopropylphenylsulfonamid, N-[(1R,2R) und (1S,2S)-2-amino-1,2-diphenylethyl]-4-chlorphenylsulfonamid, N-[(1R,2R) und (1S,2S)-2-amino-1,2-diphenylethyl]-3-chlorphenylsulfonamid, N-[(1R,2R) und (1S,2S)-2-amino-1,2-diphenylethyl]-2-chlorphenylsulfonamid, N-[(1R,2R) und (1S,2S)-2-amino-1,2-diphenylethyl]-4-fluorphenylsulfonamid, N-[(1R,2R) und (1S,2S)-2-amino-1,2-diphenylethyl]-3-fluorphenylsulfonamid, N-[(1R,2R) und (1S,2S)-2-amino-1,2-diphenylethyl]-2-fluorphenylsulfonamid, N-[(1R,2R) und (1S,2S)-2-amino-1,2-diphenylethyl]-4-methoxyphenylsulfonamid, N-[(1R,2R) und (1S,2S)-2-amino-1,2-diphenylethyl]-3-methoxyphenylsulfonamid, N-[(1R,2R) und (1S,2S)-2-amino-1,2-diphenyethyl]-2-methoxyphenylsulfonamid, N-[(1R,2R) und (1S,2S)-2-amino-1,2-diphenylethyl]-1-naphtylsulfonamid, N-[(1R,2R) und (1S,2S)-2-amino-1,2-diphenylethyl]-2-naphtylsulfonamid, N-[(1R,2R) und (1S,2S)-2-amino-1,2-diphenylethyl]-pentafluorphenylsulfonamid, N-[(1R,2R) und (1S,2S)-2-amino-1,2-diphenylethyl]-methansulfonamid, oder Mischungen der jeweiligen Enantiomere wie insbesondere die Racemate.

Das erfindungsgemäße Verfahren wird in Gegenwart von Wasser, Base und organischem Lösungsmittel durchgeführt. Geeignete organische Lösungsmittel sind beispielsweise:

Amide wie z.B. Dimethylformamid, N-Methylpyrrolidinon, gegebenenfalls halogenierte aliphatische oder aromatische Lösungsmittel mit bis zu 16 Kohlenstoffatomen wie z.B. Toluol, o-, m-, p-Xylol, Tetrachlorkohlenstoff, Chloroform, Dichlormethan, Chlorbenzol, die isomeren Dichlorbenzole, Fluorbenzol, Nitrile wie zum Beispiel Acetonitril und Benzonitril, Sulfoxide wie Dimethylsulfoxid oder Mischungen davon.

Vorteilhafterweise wählt man das organische Lösungsmittel so aus, dass das Reaktionsgemisch zwei flüssige Phasen ausbildet. Besonders geeignet sind dafür gegebenenfalls halogenierte aliphatische oder aromatische Lösungsmittel mit bis zu 16 Kohlenstoffatomen, wobei Tetrachlorkohlenstoff, Chloroform, Dichlormethan, und Chlorbenzol bevorzugt und Dichlormethan noch weiter bevorzugt sind.

Das Volumenverhältnis von Wasser zu organischem Lösungsmittel beträgt 20:1 bis 1:20.

In einer bevorzugten Ausführungsform wird das erfindungsgemäße Verfahren so durchgeführt, dass das Reaktionsgemisch zwei flüssige Phasen ausbildet und die wässrige Phase einen pH-Wert bezogen auf 25°C von 8 oder mehr, bevorzugt 11 bis 14 aufweist.

Die Einstellung des pH-Wertes erfolgt durch den Einsatz einer Base.

Als Basen eignen sich Alkali- und Erdalkalihydroxide und -carbonate, besonders bevorzugt sind Kalium- und Natriumhydroxid.

Die molare Gesamtmenge der eingesetzten Base kann beispielsweise 1 bis 10 Äquivalente bezogen auf das eingesetzte Diamin der Formel (II) betragen, bevorzugt sind 1,1 bis 3 Äquivalente. Größere Mengen an Base sind möglich aber unwirtschaftlich.

Dabei kann die Gesamtmenge Base beispielsweise von Beginn an oder zumindest teilweise im Verlauf der Reaktion zugegeben werden.

Das molare Verhältnis von Verbindungen der Formel (II) zu Verbindungen der Formel (III) beträgt 0,8 bis 1,3 Äquivalente.

Die Temperatur bei der Umsetzung beträgt -20°C bis 50°C,.

Die Reaktionsdauer kann beispielsweise 10 Minuten bis 24 Stunden, bevorzugt 30 min bis 2 h, betragen.

In Fällen, in denen die Reaktionsmischung zwei flüssige Phasen enthält, ist es besonders vorteilhaft, für eine ausreichende Durchmischung zu sorgen. Das kann beispielsweise durch intensives Rühren erfolgen.

Aus der Reaktionslösung können die Produkte beispielsweise durch Überführung in das Hydrohalogenid, Kristallisation oder Fällung des Hydrohalogenids und Freisetzung der Verbindung der Formel (I) aus dem Hydrohalogenid mit Base isoliert werden.

Die Umsetzung in das Hydrohalogenid, vorzugsweise das Hydrochlorid kann vorteilhaft durch Umsetzung der Reaktionslösung mit wässriger Halogenwasserstoffsäure, vorzugsweise wässriger Salzsäure erfolgen.

Alternativ dazu können die Verbindungen der Formel (I) auch durch Kristallisation aus einem geeigneten Lösungsmittel oder Lösungsmittelgemisch bzw. aus der Reaktionslösung oder durch Chromatographie weiter gereinigt werden.

Als besonders vorteilhaft hat es sich erwiesen, die durch das erfindungsgemäße Verfahren herstellbaren Reaktionslösungen nach Abtrennung der wässrigen Phase und gegebenenfalls Trocknung der organischen Phase direkt und ohne weitere Aufreinigung weiter einzusetzen.

Von der Erfindung sind daher auch Verfahren zur Herstellung von Lösungen enthaltend Verbindungen der Formel (I),

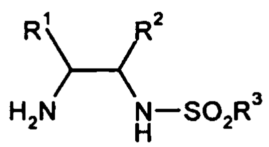

(I)

in der

$R^1$ und $R^2$ für Phenyl stehen und

$R^3$ für $C_1$-$C_4$-Alkyl, $C_1$-$C_4$-Fluoralkyl, Phenyl oder Naphthyl steht, das mit keinem, einem, zwei, drei, vier oder fünf Resten weiter substituiert sein kann, die ausgewählt sind aus der Gruppe $C_1$-$C_4$-Alkyl, $C_1$-$C_4$-Alkoxy, $C_1$-$C_4$-Fluoralkyl, Fluor und Chlor,
welches folgende Schritte umfasst:

a) Umsetzung von Verbindungen der Formel (II)

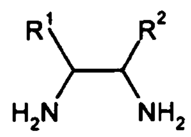

(II)

in der

$R^1$ und $R^2$ die unter der Formel (I) genannte Bedeutung besitzen

- in Gegenwart von Wasser und
- in Gegenwart von organischen Lösungsmittel,
- wobei das Volumenverhältnis von Wasser zu organischen Lösungsmittel 20:1 bis 1:20 beträgt, und
- in Gegenwart von Basen aus der Gruppe Alkali- oder Erdalkalihydroxide oder - carbonate,
- bei einer Temperatur im Bereich von -20°C bis 50°C
  mit Sulfonylhalogeniden der Formel (III) ,

$$R^3SO_2X \qquad \text{(III)}$$

in der

X für Fluor, Chlor, Brom oder Iod steht und

$R^3$ die unter der Formel (I) genannte Bedeutung besitzt,

wobei das molare Verhältnis der Verbindungen (II) zu (III) 0,8 bis 1,3 Äquivalente beträgt, und

b) zumindest teilweises Entfernen von Wasser.

Bevorzugt erfolgt die Umsetzung in einem Reaktionsgemisch, das zwei flüssige Phasen ausbildet. In diesem Fall kann das Entfernen von Wasser beispielsweise durch Abtrennung der wässrigen Phase und gegebenenfalls anschließender Trocknung der organischen Phase erfolgen.

Trocknungsmethoden für organische Phasen sind dem Fachmann hinlänglich bekannt und umfassen beispielsweise die azeotrope Destillation gegebenenfalls unter vermindertem Druck, die Trocknung über Natrium- oder Magnesiumsulfat, Molsieben und anderen gegenüber organischen Lösungsmitteln inerten Trocknungsmitteln und anschließende Filtration.

Bevorzugt weisen die erfindungsgemäßen Lösungen einen Wassergehalt von 2 Volumen-% oder weniger, bevorzugt 1 % oder weniger auf.

Die erfindungsgemäßen Lösungen enthaltend die Verbindungen der Formel(I) sowie die erfindungsgemäß herstellbaren Verbindungen der Formel (I) eignen sich insbesondere zur Anwendung in der Katalyse insbesondere der homogenen Katalyse bzw. zur Herstellung von Metallkomplexen, insbesondere Übergangsmetallkomplexen.

Bevorzugt eignen sich die erfindungsgemäßen Lösungen enthaltend die Verbindungen der Formel(I) sowie die erfindungsgemäß herstellbaren Verbindungen der Formel (I) zur Herstellung von Ruthenium-, Rhodium- oder Iridiumkomplexen und zur Anwendung in Hydrierungen, wie insbesondere zur Hydrierung von Iminen und Ketonen in Gegenwart von Ruthenium-, Rhodium- oder Iridiumkomplexen, Wasserstoffdonoren und Basen, der sogenannten Transferhydrierung. Typische Wasserstoffdonoren sind dabei beispielsweise Ameisensäure und Isopropanol, typische Basen beispielsweise Alkalimetallhydroxide und Amine wie insbesondere Triethylamin.

Eine zusammenfassende Darstellung über Transferhydrierungen als Methode zur katalytischen Reduktion geben z.B. Zassinovich et al. in Chem. Rev. 1992, 92, 1051-1069 und Noyori et al. in Acc. Chem. Res. 1997, 30, 97-102 und Wills et al. in Tetrahedron, Asymmetry, 1999, 2045.

Von der Erfindung ist weiterhin ein Verfahren zur Herstellung von Katalysatoren, welches folgende Schritte umfasst:

a) Umsetzung von Verbindungen der Formel (II)

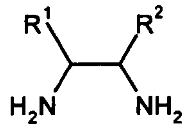

(II)

in der

$R^1$ und $R^2$ für Phenyl stehen,

- in Gegenwart von Wasser und
- in Gegenwart von organischen Lösungsmittel,

wobei das Volumenverhältnis von Wasser zu organischen Lösungsmittel 20:1 bis 1:20 beträgt, und

- in Gegenwart von Basen aus der Gruppe Alkali- oder Erdalkalihydroxide oder - carboante,
- bei einer Temperatur im Bereich von -20 bis 50°C
mit Sulfonylhalogeniden der Formel (III),

$R^3SO_2X$ (III)

in der

X für Fluor, Chlor, Brom oder Iod steht und

$R^3$ für $C_1$-$C_4$-Alkyl, $C_1$-$C_4$-Fluoralkyl, Phenyl oder Naphthyl steht, das mit keinem, einem, zwei, drei, vier oder fünf Resten weiter substituiert sein kann, die ausgewählt sind aus der Gruppe $C_1$-$C_4$-Alkyl, $C_1$-$C_4$-Alkoxy, $C_1$-$C_4$-Fluoralkyl, Fluor und Chlor,
wobei das molare Verhältnis der Verbindungen (II) zu (III) 0,8 bis 1,3 Äquivalente beträgt,

b) zumindest teilweises Entfernen von Wasser und

c) Umsetzung der nach Schritt b) erhaltenen Lösung ohne weitere Zwischenisolierung
mit Verbindungen der Formel (IV)

$[MZ_n(R^4)]_2$ (IV)

in der

M für Ruthenium, Rhodium oder Iridium,

$R^4$ für eine aromatische Verbindung mit 6 bis 12 Ringkohlenstoffatomen steht, die weiterhin mit bis zu 6 Resten substituiert sein kann, die jeweils unabhängig voneinander ausgewählt sind aus der Gruppe $C_1$-$C_8$-Alkyl, Benzyl und Phenyl, oder mit bis zu fünf Resten substituiertes Cyclopentadienyl oder Indenyl steht und

Z steht für Chlor, Brom oder Iod und

n im Falle dass M für Ruthenium steht zwei ist

n im Falle dass M für Rhodium oder Iridium steht, eins ist.
gegebenenfalls und bevorzugt in Gegenwart eines tertiären Amins.

Bevorzugt erfolgt die Umsetzung in einem Reaktionsgemisch, das zwei flüssige Phasen ausbildet. In diesem Fall kann das Entfernen von Wasser beispielsweise durch Abtrennung der wässrigen Phase und gegebenenfalls Trocknung der organischen Phase erfolgen.

Beispielsweise und bevorzugt werden solche Verbindungen der Formel (IV) eingesetzt, in denen M für Ruthenium steht und n gleich zwei ist.

Bevorzugt steht in Verbindungen der Formel (IV) $R^4$ für Benzol oder Naphthalin, das mit einem, zwei, drei, vier, fünf oder sechs Resten substituiert sein kann, die jeweils unabhängig voneinander ausgewählt sind aus der Gruppe Methyl, Ethyl, n-Propyl, Isopropyl und tert.-Butyl oder für Pentamethylcyclopentadienyl.

Bevorzugt steht in Verbindungen der Formel (IV) $R^4$ für Mesitylen, Benzol oder Cumol.

Als Verbindungen der Formel (IV) seien genannt:

Benzoldichlororuthenium-Dimer, Mesitylendichlororuthenium-Dimer, Cumoldichlororuthenium-Dimer, Pentamethylcyclopentadienylrhodiumchloro-Dimer und Pentamethylcyclopentadienyliridiumchloro-Dimer.

Im Rahmen der Erfindung ist als Verbindung der Formel (IV) Cumoldichlororuthenium-Dimer besonders bevorzugt.

In einer bevorzugten Ausführungsform beträgt das molare Verhältnis von ursprünglich eingesetzter Verbindung der Formel (II) zu Verbindungen der Formel (IV) 2:1 bis 3:1, besonders bevorzugt 2,2:1 bis 2,6:1.

Bevorzugt eignen sich die erfindungsgemäßen Katalysatoren zur Anwendung in Hydrierungen, wie insbesondere zur Hydrierung von Iminen und Ketonen in Gegenwart von Wasserstoffdonoren und Basen, der sogenannten Transferhydrierung.

Der Vorteil der Erfindung liegt darin, dass die Verbindungen der Formel (I) in hoher Ausbeute und sehr guter Selektivität erhalten werden können. Weiterhin wurde gefunden, dass die Produkte der Sulfonylierung ohne Zwischenisolierung für die Herstellung von Katalysatoren eingesetzt werden können, was eine erhebliche Vereinfachung in der technischen Anwendung darstellt.

## Beispiele

### Beispiel 1

In einem 3-Halskolben werden 5,0 g S,S-Diphenylethylendiamin in 40 ml $CH_2Cl_2$ gelöst. Anschließend werden 40 ml 1 M Natronlauge zugegeben und mittels Eisbad gekühlt. Danach wird innerhalb von 60 min eine Lösung aus 4,49 g p-Toluolsulfonylchlorid in 80 mL $CH_2Cl_2$, bei 0°C eingetropft. Nach 1 h bei 0°C wird die Reaktion abgebrochen. Die organische Phase wird abgetrennt, mit Wasser gewaschen und das Lösungsmittel am Rotationsverdampfer entfernt.
Rohprodukt-Selektivität Diamin:Mono:Disubstitutionsprodukt: 3,5:93:3,5
Es wird aus Toluol : Hexan kristallisiert.
Ausbeute: 7,8 g kristallines Produkt (91 %), Selektivität: Mono: Disubstitutionsprodukt: 95,5:4,5.

### Beispiel 2 (zum Vergleich)

In einer 3-Halskolben werden 2.21 g p-Toluolsulfonylchlorid in 40 ml $CH_2Cl_2$und mit 3,0 ml Triethylamin (1,8 eq) versetzt. Es wird auf 0°C gekühlt und 2,46 g S,S-Diphenyethylendiamin werden zugeben. Nach 1 h bei 0°C und 4 h bei RT wird die Reaktion aufgearbeitet. Es wird mit 50 ml Dichlormethan verdünnt und mit 50 ml 0,5 M NaOH gewaschen, die trübe organische Phase abgetrennt, mit 20 ml Wasser und 50 ml NaCl-Lösung gewaschen und das Lösungsmittel am Rotationsverdampfer entfernt. Es wird mit Toluol versetzt und restliches Triethylamin azeotrop entfernt.

Ausbeute: 2,93 g ("69 %") Rohprodukt, Selektivität: Mono: Disubstitutionsprodukt: 83:17.

### Beispiel 3

In einer 3-Halskolben werden 2,0 g S,S-Diphenyethylendiamin in 20 ml $CH_2Cl_2$ gelöst. Anschließend werden 10 ml 2 M Natronlauge zugegeben und mittels Eisbad gekühlt. Danach wird innerhalb von 20 min eine Lösung aus 2,08

g Mesitylsulfonylchlorid in 20 ml $CH_2Cl_2$ bei 0°C eingetropft. Nach 1 h bei 0°C wird die Reaktion aufgearbeitet. Die organische Phase wird abgetrennt, mit Wasser und Natriumchlorid-Lösung gewaschen und das Lösungsmittel am Rotationsverdampfer entfernt. Der Rückstand von 3,9 g zeigt eine Selektivität: Diamin:Mono:Disubstitutionsprodukt: 1,7: 92:6,5.
Rekristallisation aus Toluol/Hexan ergibt 3,1 g kristallines Produkt (83 %), Selektivität: Mono:Disubstitutionsprodukt: 94,8:5,2.

**Beispiel 4 (zum Vergleich)**

In einer 3-Halskolben werden 2,0 g S,S-Diphenyethylendiamin in 20 ml $CH_2Cl_2$ gelöst, 1,98 ml $Et_3N$ (1,5 eq) zugegeben und auf 0°C gekühlt. Es wird eine Lösung von 2,08 g Mesitylsulfonylchlorid in 20 mL $CH_2Cl_2$ bei dieser Temperatur innerhalb von 20 min zugetropft. Es wird innerhalb von einer Stunde auf Raumtemperatur aufgetaut. Es wird mit 20 ml Wasser und 50 ml NaCl-Lösung gewaschen und das Lösungsmittel am Rotationsverdampfer entfernt. Es wird mit Toluol versetzt und restliches Triethylamin azeotrop entfernt.

Selektivität: Mono:Disubstitutionsprodukt 84:16.

**Beispiel 5**

In einer 3-Halskolben werden 2,0 g S,S-Diphenyethylendiamin in 20 ml $CH_2Cl_2$ gelöst. Anschließend werden 10 ml 2 M Natronlauge zugegeben und mittels Eisbad gekühlt. Danach wird innerhalb von 20 min eine Lösung aus 1,68 g Phenylsulfonylchlorid in 20 ml $CH_2Cl_2$, bei 0°C eingetropft. Nach 1 h bei 0°C wird die Reaktion für 12 h bei Raumtemperatur weitergerührt. Die organische Phase wird abgetrennt, mit Wasser und Natriumchlorid-Lösung gewaschen und das Lösungsmittel am Rotationsverdampfer entfernt. Der Rückstand von 3,1 g zeigt eine Selektivität: Diamin:Mono: Disubstitutionsprodukt: 2:94:4.
Rekristallisation aus Toluol:Hexan ergibt 2,9 g kristallines Produkt (87 %), Selektivität: Mono:Disubstitutionsprodukt: 95:5.

**Beispiel 6 (zum Vergleich)**

In einer 3-Halskolben werden 2,0 g S,S-Diphenyethylendiamin in 20 ml $CH_2Cl_2$ gelöst, 1,98 ml $Et_3N$ (1,5 eq) zugegeben und auf 0°C gekühlt. Es wird eine Lösung von 1,68 g Phenylsulfonylchlorid in 20 ml $CH_2Cl_2$ bei dieser Temperatur innerhalb von 20 min zugetropft. Es wird innerhalb von einer Stunde auf RT aufgetaut und weitere 12 h bei Raumtemperatur gerührt. Es wird mit 20 ml Wasser und 50 ml NaCl-Lösung gewaschen und das Lösungsmittel am Rotationsverdampfer entfernt. Es wird mit Toluol versetzt und restliches Triethylamin azeotrop entfernt.

Selektivität: Mono:Disubstitutionsprodukt: 91:9.

**Beispiel 7 (zum Vergleich)**

In einer 3-Halskolben werden 1,0 g S,S-Diphenyethylendiamin in 20 ml $CH_2Cl_2$ gelöst. Anschließend werden 10 ml 1 M Natronlauge zugegeben und mittels Eisbad gekühlt. Danach wird innerhalb von 20 min eine Lösung aus 0,70 Pentafluorphenylsulfonylchlorid in 20 ml $CH_2Cl_2$, bei 0°C eingetropft. Nach 1 h bei 0°C wird die Reaktion für 12 h bei Raumtemperatur weitergerührt. Die organische Phase wird abgetrennt, mit Wasser und Natriumchlorid-Lösung gewaschen und das Lösungsmittel am Rotationsverdampfer entfernt.
Es werden 1,5 g kristallines Produkt (72 %) isoliert, Selektivität: Mono:Disubstitutionsprodukt: 83:17.

**Beispiel 8 (zum Vergleich)**

In einer 3-Halskolben werden 1,0 g S,S-Diphenyethylendiamin in 20 ml $CH_2Cl_2$ gelöst. 0,99 ml $Et_3N$ (1,5 eq) zugegeben und auf 0°C gekühlt. Es wird eine Lösung von 1,68 g Phenylsulfonylchlorid in 20 ml $CH_2Cl_2$ bei dieser Temperatur innerhalb von 20 min zugetropft. Es wird innerhalb von einer Stunde auf RT aufgetaut und weitere 12 h bei Raumtemperatur gerührt. Es wird mit 20 ml Wasser und 50 ml NaCl-Lösung gewaschen und das Lösungsmittel am Rotationsverdampfer entfernt. Es wird mit Toluol versetzt und restliches Triethylamin azeotrop entfernt.

Selektivität: Mono:Disubstitutionsprodukt: 76:24.

**Beispiel 9**

Herstellung einer Katalysatorstammlösung in situ ohne Isolierung des Liganden

In einem Schlenkkolben werden 0,4 g S,S-Diphenylethylendiamin in 10 ml $CH_2Cl_2$ gelöst. Anschließend werden 4 ml 1 M Natronlauge zugegeben und mittels Eisbad gekühlt. Danach wird innerhalb von 10 min. unter starkem Rühren

eine Lösung aus 0,36 g Tosylchlorid in 10 ml $CH_2Cl_2$, bei 0°C eingetropft. Nach 1 h bei 0°C werden die Phasen getrennt. Die organische Phase wird abgetrennt, mit Wasser und NaCl-Lösung gewaschen.
Die so hergestellte Ligandlösung wird entgast und in einem Schlenkgefäß mit 0,56 g $[(Cumol)RuCl_2]_2$ (= 0,44 Mol-Äquivalente bezogen auf das S,S-Diphenylethylendiamin) und 550 μl $Et_3N$ für 20 min gerührt.

**Beispiel 10**

Die Hälfte der in Beispiel 9 hergestellten Katalysator-Lösung wird für diesen Versuch eingesetzt.
In einem 500 ml-Vierhalskolben mit Begasungs-Rührer, Rückflusskühler und Thermometer wird eine Ameisensäure/$Et_3N$-Mischung aus 70 ml Triethylamin und 20 ml HCOOH durch langsames Zutropfen innerhalb von 5 min per Tropftrichter von HCOOH zum $Et_3N$ unter Rührung und Eiskühlung hergestellt. Zu diesem zweiphasigen Gemisch werden 76 g Methyl-3-keto-3-(2-thiophen-yl)propanoat (S/C = 500) gegeben, die homogene gelbe Lösung mit 50 ml Dichlormethan versetzt und die Gesamtmischung durch Durchleiten von Argon für 20 min. entgast. Es wird auf 40°C temperiert und unter starkem Rühren die Hälfte der Katalysatorlösung aus Beispiel 5 auf einmal zum Reaktionsansatz per Spritze geben. Es wird unter Argondurchleitung für 18 h gerührt, dann die Reaktionslösung mit Wasser und $CH_2Cl_2$ verdünnt, für 10 min nachgerührt, nach Phasentrennung die $H_2O$ Phase 2 x mit $CH_2Cl_2$ extrahiert. Die vereinigten org. Phasen werden mit Wasser und NaCl-Lösung gewaschen, über $MgSO_4$ getrocknet, filtriert und dann das Lösungsmittel am Rotationsverdampfer entfernt. Es werden 75 g S-Methyl-3-hydroxy-3-(2-thiophen-yl)propanoat erhalten (97,7 %).
Umsatz (GC): 99,9 %. Ee (Chiral-GC, IVADEX 3): 97,2 %.

**Beispiel 11 (zum Vergleich)**

Versuchsdurchführung wie Beispiel 10, allerdings werden als Katalysator unabhängig präparierte 0,52 g [N-[(1R,2R)-2-(amino-mN)-1,2-diphenylethyl]-p-tolylsulfontolylsulfonamidato-κN]chloro[($\eta^6$)-cumol]-ruthenium(II) eingesetzt. Es wird für 18 h gerührt und nachfolgend aufgearbeitet wie in Beispiel 6 beschrieben. Es werden 75 g R-Methyl-3-hydroxy-3-(2-thiophen-yl)propanoat erhalten (97,7 %).
Umsatz: 99,5 %. ee: 96,9 %.

**Beispiel 12**

In einem 250 ml-Vierhalskolben mit Begasungs-Rührer, Rückflusskühler und Thermometer wird eine Ameisensäure/$Et_3N$-Mischung aus 41 ml Triethylamin und 12 ml HCOOH durch langsames Zutropfen innerhalb von 5 min per Tropftrichter von HCOOH zum $Et_3N$ unter Rührung und Eiskühlung hergestellt. Zu diesem zweiphasigen Gemisch werden 49,5 g 4-Bromacetophenon (S/C = 300) gegeben, die homogene gelbe Lösung mit 30 ml Dichlormethan versetzt und die Gesamtmischung durch Durchleiten von Argon für 20 min entgast. Es bei Raumtemperatur und unter starkem Rühren die Hälfte der Katalysatorlösung aus Beispiel 9 auf einmal zum Reaktionsansatz per Spritze geben. Es wird unter Argondurchleitung für 18 h gerührt, nachfolgend mit Wasser und $CH_2Cl_2$ verdünnt, für 10 min nachgerührt, nach Phasentrennung die $H_2O$ Phase 2 x mit $CH_2Cl_2$ extrahiert. Die vereinigten org. Phasen werden mit NaCl-Lösung gewaschen, über $MgSO_4$ getrocknet, filtriert und dann das Lösungsmittel am Rotationsverdampfer entfernt. Der Umsatz beträgt 99,5 % (GC). ee (Chiral-GC): 93,9 %.
Der ölige viskose Rückstand wird mit wenig Hexan vermischt und bei -20°C kristallisiert. Man erhält im ersten Kristallisat 23 g (46 %) des weißen kristallinen Produktes p-Brom-1-phenylethan-1-ol (ee: >99 %).

**Beispiel 13 (zum Vergleich)**

Die Durchführung erfolgt analog zu Beispiel 11, mit dem Unterschied, dass als Substrat 4,98 g 4-Bromacetophenon und als Katalysator 31 mg [N-[(1R,2R)-2-(amino-κN)-1,2-diphenylethyl]-p-tolylsulfonamidato-κN]chloro[($\eta^6$)-1,3,5-trimethylbenzol]-ruthenium(II) eingesetzt werden.
Es werden nach 48 h Reaktionszeit (nicht optimiert) >99 % Umsatz erreicht. Das Rohprodukt hat einen Enantiomerenüberschuss von 95,3 %. Durch Kristallisation bei -20 °C aus Hexan gelingt die Aufreinigung auf >99 % Enantiomerenüberschuss.

**Beispiel 14**

In einem 250 ml-Vierhalskolben mit Begasungs-Rührer, Rückflusskühler und Thermometer wird eine Ameisensäure/$Et_3N$-Mischung aus 41 ml Triethylamin und 12 ml HCOOH durch langsames Zutropfen innerhalb von 5 min per Tropftrichter von HCOOH zum $Et_3N$ unter Rührung und Eiskühlung hergestellt. Zu diesem zweiphasigen Gemisch werden 49,5 g 2-Bromacetophenon (S/C = 300) gegeben, die homogene gelbe Lösung mit 30 ml Dichlormethan versetzt

und die Gesamtmischung durch Durchleiten von Argon für 20 min entgast. Es wird auf 25°C temperiert und unter starkem Rühren eine analog zu Beispiel 9 (halber Ansatz) hergestellte Katalysatorlösung auf einmal zum Reaktionsansatz per Spritze geben. Es wird unter Argon für 18 h gerührt, mit Wasser und $CH_2Cl_2$ verdünnt, für 10 min nachgerührt, nach Phasentrennung die $H_2O$ Phase 2 x mit $CH_2Cl_2$ extrahiert. Die vereinigten org. Phasen werden mit NaCl-Lösung gewaschen, über $MgSO_4$ getrocknet, filtriert und dann das Lösungsmittel am Rotationsverdampfer entfernt. Der Umsatz beträgt 99,8 % (GC). ee (Chiral-GC): 92,6 %.
Der Rückstand wird in Hexan gelöst und bei -20°C kristallisiert. Man erhält im ersten Kristallisat 35 g (70 %) des weißen kristallinen Produktes 2-Brom-1-phenylethan-1-ol (ee: >99,5 %).

## Patentansprüche

1. Verfahren zur Herstellung von Verbindungen der Formel (I),

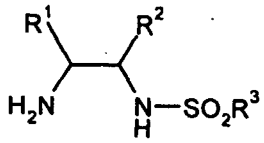

(I)

in der

$R^1$ und $R^2$ für Phenyl stehen und
$R^3$ für $C_1$-$C_4$-Alkyl, $C_1$-$C_4$-Fluoralkyl, Phenyl oder Naphthyl steht, das mit keinem, einem, zwei, drei, vier oder fünf Resten weiter substituiert sein kann, die ausgewählt sind aus der Gruppe $C_1$-$C_4$-Alkyl, $C_1$-$C_4$-Alkoxy, $C_1$-$C_4$-Fluoralkyl, Fluor und Chlor,
**dadurch gekennzeichnet, dass**
Diamine der Formel (II)

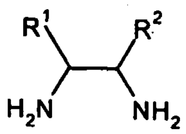

(II)

in der
$R^1$ und $R^2$ die unter der Formel (I) genannte Bedeutung besitzen

- in Gegenwart von Wasser und
- in Gegenwart von organischem Lösungsmittel,

wobei und Volumenverhältnis von Wasser in organischen Lösungsmittel 20:1 bis 1:20 beträgt, und

- in Gegenwart von Basen aus der Gruppe Alkali- oder Erdalkalihydroxide oder
- carbonate
- bei einer Temperatur im Bereich von -20°C bis 50°C

mit Sulfonylhalogeniden der Formel (III) umgesetzt werden,

$R^3SO_2X$ (III)

in der

X für Fluor, Chlor, Brom oder Iod steht und
$R^3$ die unter der Formel (I) genannte Bedeutung besitzt,

wobei das molare Verhältnis der Verbindungen (II) zu den Verbindungen der Formel (III) 0,8 bis 1,3 Äquivalente beträgt.

2. Verfahren gemäß Anspruch 1, **dadurch gekennzeichnet, dass** X für Fluor oder Chlor steht.

3. Verfahren gemäß Ansprüchen 1 oder 2, **dadurch gekennzeichnet, dass** chirale Verbindungen der Formel (II) eingesetzt werden, die eine optische Reinheit von 80 % ee oder mehr aufweisen.

4. Verfahren gemäß einem oder mehreren der Ansprüche 1 bis 3, **dadurch gekennzeichnet, dass** als organische Lösungsmittel eingesetzt werden:

   Amide, gegebenenfalls halogenierte aliphatische oder aromatische Lösungsmittel mit bis zu 16 Kohlenstoffatomen, Nitrile, Sulfoxide oder Mischungen davon.

5. Verfahren gemäß einem oder mehreren der Ansprüche 1 bis 4, **dadurch gekennzeichnet, dass** als organische Lösungsmittel eingesetzt werden:

   Tetrachlorkohlenstoff, Chloroform, Dichlormethan oder Chlorbenzol.

6. Verfahren gemäß einem oder mehreren der Ansprüche 1 bis 5, **dadurch gekennzeichnet, dass** das Reaktionsgemisch zwei flüssige Phasen ausbildet und die wässrige Phase einen pH-Wert bezogen auf 25°C von 8 oder mehr aufweist.

7. Verfahren gemäß einem oder mehreren der Ansprüche 1 bis 6, **dadurch gekennzeichnet, dass** aus der Reaktionslösung die Produkte beispielsweise durch Überführung in das Hydrohalogenid, Kristallisation oder Fällung des Hydrohalogenids und Freisetzung der Verbindung der Formel (I) aus dem Hydrohalogenid mit Base isoliert werden.

8. Verfahren zur Herstellung von Lösungen enthaltend Verbindungen der Formel (I),

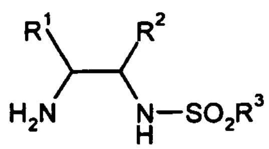

(I)

in der

$R^1$ und $R^2$ für Phenyl stehen und
$R^3$ für $C_1$-$C_4$-Alkyl, $C_1$-$C_4$-Fluoralkyl, Phenyl oder Naphthyl steht, das mit keinem, einem, zwei, drei, vier oder fünf Resten weiter substituiert sein kann, die ausgewählt sind aus der Gruppe $C_1$-$C_4$-Alkyl, $C_1$-$C_4$-Alkoxy, $C_1$-$C_4$-Fluoralkyl, Fluor und Chlor,

welches folgende Schritte umfasst:

a) Umsetzung von Verbindungen der Formel (II)

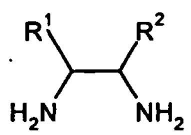

(II)

in der

$R^1$ und $R^2$ die unter der Formel (I) genannte Bedeutung besitzen

- in Gegenwart von Wasser und
- in Gegenwart von organischen Lösungsmittel,
- wobei das Volumenverhältnis von Wasser zu organischen Lösungsmittel 20:1 bis 1:20 beträgt, und
- in Gegenwart von Basen aus der Gruppe Alkali- oder Erdalkalihydroxide oder -carbonate,
- bei einer Temperatur im Bereich von -20°C bis 50°C

mit Sulfonylhalogeniden der Formel (III),

$$R^3SO_2X \qquad (III)$$

in der

X für Fluor, Chlor, Brom oder Iod steht und
$R^3$ die unter der Formel (I) genannte Bedeutung besitzt,

wobei das molare Verhältnis der Verbindungen (II) zu (III) 0,8 bis 1,3 Äquivalente beträgt, und
b) zumindest teilweises Entfernen von Wasser.

**9.** Verfahren zur Herstellung von Katalysatoren, welches folgende Schritte umfasst:

a) Umsetzung von Verbindungen der Formel (II)

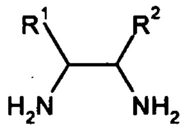

(II)

in der

$R^1$ und $R^2$ für Phenyl stehen,

- in Gegenwart von Wasser und
- in Gegenwart von organischen Lösungsmittel,

wobei das Volumenverhältnis von Wasser zu organischen Lösungsmittel 20:1 bis 1:20 beträgt, und

- in Gegenwart von Basen aus der Gruppe Alkali- oder Erdalkalihydroxide oder -carbonate,
- bei einer Temperatur im Bereich von -20 bis 50°C

mit Sulfonylhalogeniden der Formel (III),

$$R^3SO_2X \qquad (III)$$

in der

X für Fluor, Chlor, Brom oder Iod steht und
$R^3$ für $C_1$-$C_4$-Alkyl, $C_1$-$C_4$-Fluoralkyl, Phenyl oder Naphthyl steht, das mit keinem, einem, zwei, drei, vier oder fünf Resten weiter substituiert sein kann, die ausgewählt sind aus der Gruppe $C_1$-$C_4$-Alkyl, $C_1$-$C_4$-Alkoxy, $C_1$-$C_4$-Fluoralkyl, Fluor und Chlor,

wobei das molare Verhältnis der Verbindungen (II) zu (III) 0,8 bis 1,3 Äquivalente beträgt,
b) zumindest teilweises Entfernen von Wasser und
c) Umsetzung der nach Schritt b) erhaltenen Lösung ohne weitere Zwischenisolierung
mit Verbindungen der Formel (IV)

$[MZ_n(R^4)]_2$ (IV)

in der

M für Ruthenium, Rhodium oder Iridium,
$R^4$ für eine aromatische Verbindung mit 6 bis 12 Ringkohlenstoffatomen steht, die weiterhin mit bis zu 6 Resten substituiert sein kann, die jeweils unabhängig voneinander ausgewählt sind aus der Gruppe $C_1$-$C_8$-Alkyl, Benzyl und Phenyl, oder mit bis zu fünf Resten substituiertes Cyclopentadienyl oder Indenyl steht und
Z für Chlor, Brom oder Iod steht und
n im Falle dass M für Ruthenium steht zwei ist
n im Falle dass M für Rhodium oder Iridium steht, eins ist.

**10.** Verfahren gemäß Anspruch 9, **dadurch gekennzeichnet, dass** Z für Chlor steht.

**11.** Verfahren zur Herstellung von Katalysatoren gemäß Anspruch 9, **dadurch gekennzeichnet, dass** solche Verbindungen der Formel (IV) eingesetzt werden, in denen M für Ruthenium steht und n gleich zwei ist.

**12.** Hydrierverfahren, welches die Herstellung des Katalysators gemäß Anspruch 9 und die Verwendung des so hergestellten Katalysators bei der Hydrierung umfasst.

## Claims

**1.** Process for preparing compounds of the formula (I),

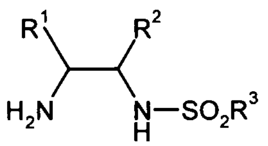

(I)

where

$R^1$ and $R^2$ are phenyl and
$R^3$ is $C_1$-$C_4$-alkyl, $C_1$-$C_4$-fluoroalkyl, phenyl or naphthyl, each of which may be further substituted by no, one, two, three, four or five radicals which are selected from the group of $C_1$-$C_4$-alkyl, $C_1$-$C_4$-alkoxy, $C_1$-$C_4$-fluoroalkyl, fluorine and chlorine,
**characterized in that**
diamines of the formula (II)

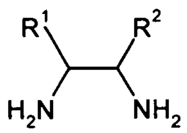

(II)

where
$R^1$ and $R^2$ are as defined under formula (I)
are reacted

- in the presence of water and
- in the presence of organic solvents,

where the volume ratio of water in organic solvents is 20:1 to 1:20, and

- in the presence of bases from the group of alkali metal or alkaline earth metal hydroxides or carbonates,
- at a temperature in the range from -20°C to 50°C,

with sulphonyl halides of the formula (III)

$$R^3SO_2X \qquad (III)$$

where
X is fluorine, chlorine, bromine or iodine and
$R^3$ is as defined under formula (I),
where the molar ratio of compounds (II) to the compounds of the formula (III) is 0.8 to 1.3 equivalents.

**2.** Process according to Claim 1, **characterized in that** X is fluorine or chlorine.

**3.** Process according to Claim 1 or 2, **characterized in that** the chiral compounds of the formula (II) are used which have an optical purity of 80% ee or more.

**4.** Process according to one or more of Claims 1 to 3, **characterized in that** the organic solvent used is:

an amide, an optionally halogenated aliphatic or aromatic solvent having up to 16 carbon atoms, a nitrile, a sulphoxide or a mixture thereof.

**5.** Process according to one or more of Claims 1 to 4, **characterized in that** the organic solvent used is:

carbon tetrachloride, chloroform, dichloromethane or chlorobenzene.

**6.** Process according to one or more of Claims 1 to 5, **characterized in that** the reaction mixture forms two liquid phases and the aqueous phase has a pH at 25°C of 8 or more.

**7.** Process according to one or more of Claims 1 to 6, **characterized in that** the products are isolated from the reaction solution, for example, by conversion into the hydrohalide, crystallization or precipitation of the hydrohalide and release of the compound of the formula (I) from the hydrohalide using base.

**8.** Process for preparing solutions comprising compounds of the formula (I)

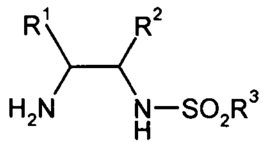

(I)

where

$R^1$ and $R^2$ are phenyl and
$R^3$ is $C_1$-$C_4$-alkyl, $C_1$-$C_4$-fluoroalkyl, phenyl or naphthyl, each of which may be further substituted by no, one, two, three, four or five radicals which are selected from the group of $C_1$-$C_4$-alkyl, $C_1$-$C_4$-alkoxy, $C_1$-$C_4$-fluoroalkyl, fluorine and chlorine,
which comprises the following steps:

a) reacting compounds of the formula (II)

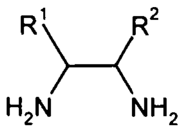

(II)

where

$R^1$ and $R^2$ are as defined under formula (I)

- in the presence of water and
- in the presence of organic solvents,
- where the volume ratio of water in organic solvents is 20:1 to 1:20, and
- in the presence of bases from the group of alkali metal or alkaline earth metal hydroxides or carbonates,
- at a temperature in the range from -20°C to 50°C,

with sulphonyl halides of the formula (III)

$R^3SO_2X$ (III)

where
X is fluorine, chlorine, bromine or iodine and
$R^3$ is as defined under formula (I),
where the molar ratio of the compounds (II) to (III) is 0.8 to 1.3 equivalents, and

b) at least partly removing water.

**9.** Process for preparing catalysts which comprises the following steps:

a) reacting compounds of the formula (II)

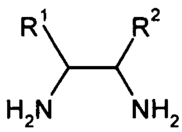

(II)

where

$R^1$ and $R^2$ are phenyl,

- in the presence of water and
- in the presence of organic solvents,

where the volume ratio of water to organic solvents is 20:1 to 1:20, and

- in the presence of bases from the group of alkali metal or alkaline earth metal hydroxides or carbonates,
- at a temperature in the range from -20 to 50°C,

with sulphonyl halides of the formula (III)

$R^3SO_2X$ (III)

where

X is fluorine, chlorine, bromine or iodine and
$R^3$ is $C_1$-$C_4$-alkyl, $C_1$-$C_4$-fluoroalkyl, phenyl or naphthyl, each of which may be further substituted by no, one, two, three, four or five radicals which are selected from the group of $C_1$-$C_4$-alkyl, $C_1$-$C_4$-alkoxy, $C_1$-$C_4$-fluoroalkyl, fluorine and chlorine,
where the molar ratio of the compounds (II) to (III) is 0.8 to 1.3 equivalents,

b) at least partly removing water
c) reacting the solution obtained in step b) without further intermediate isolation
with compounds of the formula (IV)

$$[MZ_n(R^4)]_2 \qquad (IV)$$

where

M is ruthenium, rhodium or iridium,
$R^4$ is an aromatic compound which has 6 to 12 ring carbon atoms and may be substituted by up to 6 radicals which are each independently selected from the group of $C_1$-$C_8$-alkyl, benzyl and phenyl, or cyclopentadienyl or indenyl, each of which are substituted by up to five radicals, and
Z is chlorine, bromine or iodine, and
n is two where M is ruthenium,
n is one where M is rhodium or iridium.

**10.** Process according to Claim 9, **characterized in that** Z is chlorine.

**11.** Process for preparing catalysts according to Claim 9, **characterized in that** the compounds of the formula (IV) used are those in which M is ruthenium and n is two.

**12.** Hydrogentatoin process which comprises the preparation of the catalyst according to Claim 9 and the use of the catalyst thus prepared.

**Revendications**

**1.** Procédé pour la préparation de composés de formule (I),

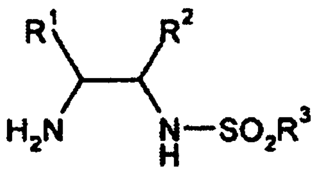

(I)

dans laquelle

$R^1$ et $R^2$ représentent le groupe phényle et
$R^3$ représente un groupe alkyle en $C_1$-$C_4$, fluoroalkyle en $C_1$-$C_4$, phényle ou naphtyle, qui peut être non substitué ou substitué en outre par un, deux, trois, quatre ou cinq radicaux qui sont choisis dans l'ensemble constitué par les groupes alkyle en $C_1$-$C_4$ , alcoxy en $C_1$-$C_4$, fluoroalkyle en $C_1$-$C_4$ et les atomes de fluor et de chlore,
**caractérisé en ce qu'**on fait réagir
des diamines de formule (II)

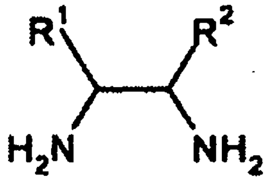

(II)

dans laquelle
$R^1$ et $R^2$ ont les significations données sous la formule (I)

- en présence d'eau et
- en présence d'un solvant organique, le rapport en volume de l'eau au solvant organique allant de 20:1 à 1:20, et
- en présence de bases choisies dans le groupe des hydroxydes ou carbonates de métaux alcalins ou alcalino-terreux,
- à une température dans la plage de -20 °C à 50°C,

avec des halogénures de sulfonyle de formule (III),

$R^3SO_2X$ (III)

dans laquelle

X représente un atome de fluor, chlore, brome ou iode, et
$R^3$ a la signification donnée sous la formule (I),
le rapport molaire des composés (II) aux composés de formule (III) étant de 0,8 à 1,3 équivalent.

2. Procédé selon la revendication 1, **caractérisé en ce que** X représente un atome de fluor ou de chlore.

3. Procédé selon la revendication 1 ou 2, **caractérisé en ce qu'**on utilise des composés chiraux de formule (II) qui présentent une pureté optique de 80 % ee (excès d'énantiomère) ou plus.

4. Procédé selon une ou plusieurs des revendications 1 à 3, **caractérisé en ce qu'**on utilise comme solvants organiques :

   des amides, des solvants aliphatiques ou aromatiques éventuellement halogénés ayant jusqu'à 16 atomes de carbone, des nitriles, des sulfoxydes ou des mélanges de ceux-ci.

5. Procédé selon une ou plusieurs des revendications 1 à 4, **caractérisé en ce qu'**on utilise comme solvants organiques:

   le tétrachlorure de carbone, le chloroforme, le dichlorométhane ou le chlorobenzène.

6. Procédé selon une ou plusieurs des revendications 1 à 5, **caractérisé en ce que** le mélange réactionnel forme deux phase liquides et la phase aqueuse présente un pH de 8 ou plus, à 25 °C.

7. Procédé selon une ou plusieurs des revendications 1 à 6, **caractérisé en ce qu'**on isole les produits à partir de la solution réactionnelle, par exemple, par conversion en l'halogénhydrate, cristallisation ou précipitation de l'halogénhydrate et libération du composé de formule (I) à partir de l'halogénhydrate à l'aide d'une base.

8. Procédé pour la préparation de solutions contenant des composés de formule (I)

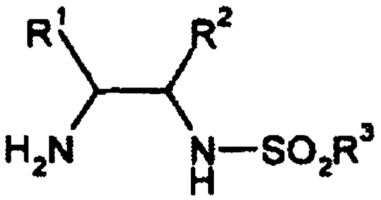

(I)

dans laquelle

$R^1$ et $R^2$ représentent le groupe phényle et
$R^3$ représente un groupe alkyle en $C_1$-$C_4$, fluoroalkyle en $C_1$-$C_4$ , phényle ou naphtyle, qui peut être non substitué ou substitué encore par un, deux, trois, quatre ou cinq radicaux qui sont choisis dans l'ensemble constitué par les groupes alkyle en $C_1$-$C_4$ , alcoxy en $C_1$-$C_4$, fluoroalkyle en $C_1$-$C_4$ et les atomes de fluor et de chlore,
qui comprend les étapes suivantes :

a) mise en réaction de composés de formule (II)

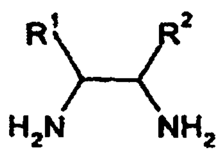

(II)

dans laquelle

$R^1$ et $R^2$ ont les significations données sous la formule (I)

- en présence d'eau et
- en présence de solvants organiques,
le rapport en volume de l'eau aux solvants organiques allant de 20:1 à 1:20, et
- en présence de bases choisies dans le groupe des hydroxydes ou carbonates de métaux alcalins ou alcalino-terreux,
- à une température dans la plage de -20 °C à 50 °C,

avec des halogénures de sulfonyle de formule (III),

$R^3SO_2X$ (III)

dans laquelle
X représente un atome de fluor, chlore, brome ou iode, et
$R^3$ a la signification donnée sous la formule (I),
le rapport molaire des composés (II) aux composés de formule (III) étant de 0,8 à 1,3 équivalent, et

b) élimination au moins partielle de l'eau.

**9.** Procédé pour la préparation de catalyseurs, qui comprend les étapes suivantes :

a) mise en réaction de composés de formule (II)

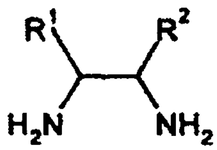

(II)

dans laquelle

$R^1$ et $R^2$ représentent le groupe phényle,

- en présence d'eau et
- en présence de solvants organiques,
le rapport en volume de l'eau aux solvants organiques allant de 20:1 à 1:20, et
- en présence de bases choisies dans le groupe des hydroxydes ou carbonates de métaux alcalins ou alcalino-terreux,
- à une température dans la plage de -20 °C à 50 °C,
avec des halogénures de sulfonyle de formule (III),

$R^3SO_2X$ (III)

dans laquelle

X représente un atome de fluor, chlore, brome ou iode, et
$R^3$ représente un groupe alkyle en $C_1$-$C_4$, fluoroalkyle en $C_1$-$C_4$, phényle ou naphtyle, qui peut être non substitué ou substitué encore par un, deux, trois, quatre ou cinq radicaux qui sont choisis dans l'ensemble constitué par les groupes alkyle en $C_1$-$C_4$, alcoxy en $C_1$-$C_4$, fluoroalkyle en $C_1$-$C_4$ et les atomes de fluor et de chlore,
le rapport molaire des composés (II) aux composés (III) étant de 0,8 à 1,3 équivalent, et

b) élimination au moins partielle de l'eau et
c) mise en réaction de la solution obtenue après l'étape b), sans autre isolement intermédiaire avec des composés de formule (IV)

$[MZ_n(R^4)]_2$ (IV)

dans laquelle

M représente le ruthénium, le rhodium ou l'iridium,
$R^4$ représente un composé aromatique ayant de 6 à 12 atomes de carbone formant le cycle, qui peut en outre être substitué par jusqu'à 6 radicaux qui sont choisis, indépendamment les uns des autres, dans l'ensemble constitué par les groupes alkyle en $C_1$-$C_8$, benzyle et phényle, ou un groupe cyclopentadiényle ou indényle substitué par jusqu'à cinq radicaux et
Z représente un atome de chlore, brome ou iode et
n est deux lorsque M représente le ruthénium
n est un lorsque M représente le rhodium ou l'iridium.

**10.** Procédé selon la revendication 9, **caractérisé en ce que** Z représente un atome de chlore.

**11.** Procédé pour la préparation de catalyseurs selon la revendication 9, **caractérisé en ce qu'**on utilise les composés de formule (IV) dans lesquels M représente le ruthénium et n est égal à deux.

**12.** Procédé d'hydrogénation, qui comprend la préparation du catalyseur selon la revendication 9 et l'utilisation du catalyseur ainsi préparé dans l'hydrogénation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1174426 A


### In der Beschreibung aufgeführte Nicht-Patentliteratur


- **Noyori et al.** *J. Amer. Chem. Soc.,* 1995, vol. 117, 7562
- **R.A. Sheldon et al.** *Eur. J. Org. Chem.,* 1999, 2315
- **BALSELLS, JAUME et al.** *J. ORG. CHEM.,* 2000, vol. 65, 5005-5008
- **BALSELLS, JAUME et al.** *TETRAHEDON-ASYMMETRY,* 1998, vol. 9, 4135-4142
- Chemistry of Opium Alkaloids, 45. Improvements in the Total Synthesis of Morphine. **MEUZELAAR G et al.** EUROPEAN JOURNAL OF ORGANIC CHEMISTRY. WILEY-VCH VERLAG, 1999, vol. 9, 2315-2321
- **Zassinovich et al.** *Chem. Rev.,* 1992, vol. 92, 1051-1069
- **Noyori et al.** *Acc. Chem. Res.,* 1997, vol. 30, 97-102
- **Wills et al.** *Tetrahedron, Asymmetry,* 1999, 2045